# NEUE EUROPÄISCHE PATENTSCHRIFT

(11) **EP 1 275 640 B2**
(45) Veröffentlichungstag und Bekanntmachung des Hinweises auf die Entscheidung über den Einspruch: **27.05.2015**
(45) Hinweis auf die Patenterteilung: 19.12.2007
(21) Anmeldenummer: 02014039.8
(22) Anmeldetag: 28.06.2002
(51) Int. Cl.: C07C 263/10, C07C 265/14

(54) **Verfahren zur Herstellung von (cyclo)aliphatischen Isocyanaten**
Process for the preparation of (cyclo)aliphatic isocyanates
Procédé de préparation d'isocyanates (cyclo)aliphatiques

(30) Priorität: 11.07.2001 DE 10133728
(43) Veröffentlichungstag der Anmeldung: 15.01.2003
(73) Patentinhaber: Bayer Intellectual Property GmbH, 40789 Monheim (DE)
(72) Erfinder: Leimkühler, Hans-Joachim, Dr., 51375 Leverkusen (DE); Stutz, Herbert, Dr., 41541 Dormagen (DE); Schmidt, Helmut, 51519 Odenthal (DE)
(74) Vertreter: Davepon, Björn

(56) Entgegenhaltungen:
- EP-A- 0 289 840
- EP-A1- 0 570 799
- EP-A1- 0 593 334
- BE-B- 546 003
- FR-A- 748 683
- FR-A1- 2 723 585
- US-A- 3 507 626
- US-A- 3 591 597
- H.-J. HENZLER: 'Zur Auslegung von Strahlsaugern für einphasige Stoffsysteme' CHEM.-ING.-TECH. Bd. 54, Nr. 1, 1982, Seiten 8 - 16
- E. BALINT: 'Numerical Modelling of Injector Operation' JOURNAL OF INDUSTRIAL CHEMISTRY Bd. 27, Nr. 1, 1999, Seiten 51 - 56
- H.-P- SCHLAG ET AL.: 'Experimentelle und theoretische Untersuchungen zur Berechnung der Kennlinien vongasbetriebenen Einphaseninjektoren und Gutaufgabeinjektoren', Bd. 313, 1993, VDI VERLAG, DÜSSELDORF Seiten 1-19, 12-15, - 108-109, 114-117

## Beschreibung

Die vorliegende Erfindung betrifft ein neues Verfahren zur Herstellung von (cyclo)aliphatischen Diisocyanaten und Triisocyanaten durch Phosgenierung von (cyclo)aliphatischen Diaminen und Triaminen in der Gasphase.

Es ist bereits bekannt, dass Diamine in der Gasphase phosgeniert werden können. In der GB-A 1 165 831 wird die Phosgenierung von (cyclo-)aliphatischen Diaminen in einem mit einem mechanischen Rührer ausgestatteten Rohrreaktor beschrieben. Der Reaktor ähnelt einem Dünnschichtverdampfer, bei dem der Rührer die Gase mischt und gleichzeitig die beheizten Wände des Rohrreaktors bestreicht, um so einen Aufbau von polymerem Material an der Rohrwand zu verhindern. Die Verwendung eines schnelllaufenden Rührers beim Umgang mit ca. 300°C heißem Phosgen erfordert jedoch hohen sicherheitstechnischen Aufwand, um den Reaktor abzudichten und den Rührer in dem hochkorrosiven Medium zu lagern.

In der EP-A 0 289 840 und in der EP-A 0 749 958 ist zur Phosgenierung von (cyclo)aliphatischen Diaminen ein zylindrischer Reaktionsraum ohne sich bewegende Teile genannt, in dem man die Reaktanden unter Aufrechterhaltung einer turbulenten Strömung miteinander zur Reaktion bringt. Aufgrund der Strömungsführung kommt es zu Rückvermischungsvorgängen, in folge derer die Produkte unter Entstehung von festen Ablagerungen mit dem Diamin aus dem Edukt reagieren. Dadurch kommt es zur Verschmutzung des Reaktors und zu Verstopfungen im Gasweg.

Es wurde nun überraschenderweise gefunden, dass es möglich ist, (cyclo)aliphatische Isocyanate durch Gasphasenphosgenierung der ihnen zugrunde liegenden Amine unter Eliminierung der genannten Nachteile des Standes der Technik herzustellen, wenn man die Strömung mindestens eines der Eduktströme im Bereich der Mischung der Reaktanden beschleunigt.

Gegenstand der Erfindung ist ein Verfahren zur Herstellung von (cyclo)aliphatischen Diisocyanaten und Triisocyanate gemäß Patentanspruch 1.

Ausgangsmaterialien für das erfindungsgemäße Verfahren sind (cyclo)aliphatische Diamine und Triamine der allgemeinen Formel (II)

R - (NH₂)ₙ (II),

in welcher
- R: für einen (cyclo)aliphatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind, und
- n: für die Zahl 2 oder 3 steht.

Typische Beispiele geeigneter (cyclo-)aliphatischer Diamine sind 1,4-Diaminobutan, 1,6-Diaminohexan, 1,11-Diaminoundecan, 1,4-Diaminocyclohexan, 1-Amino-3,3,5-trimethyl-5-aminomethylcyclohexan (IPDA), 4,4'-Diaminodicyclohexylmethan oder 4,4'-Diaminodicyclohexylpropan-(2,2). Ein Beispiel für ein geeignetes (cyclo)aliphatisches Triamin ist 1,8-diamino-4-(aminomethyl)octan, Triaminononan. Bevorzugte Ausgangsamine sind 1,6-Diaminohexan, IPDA und 4,4'-Diaminodicyclohexylmethan.

Erfindungsgemäß werden (cyclo)aliphatische Diisocyanate oder (cyclo)aliphatische Triisocyanate der Formel (I),

R - (NCO)ₙ (I),

hergestellt, in welcher
- R: für einen (cyclo)aliphatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind, und
- n: für die Zahl 2 oder 3 steht.

Bevorzugt werden im erfindungsgemäßen Verfahren 1,6-Diisocyanatohexan oder 1-Isocyanato-3,3,5-trimethyl-5-isocyanatomethylcyclohexan (IPDI) hergestellt. Bevorzugtes Triisocyanat ist 1,8-Diisocyanato-4-(isocyanatomethyl)octan, Triisocyanatononan.

Die Ausgangsamine werden vor der Durchführung des erfindungsgemäßen Verfahrens verdampft und auf 200°C bis 600°C, vorzugsweise 250°C bis 500°C erhitzt und gegebenenfalls verdünnt mit einem Inertgas (z.B. N₂, Edelgase wie Ar) und/oder mit den Dämpfen eines inerten Lösungsmittels wie Dichlorbenzol dem Reaktor zugeführt.

Das bei der Phosgenierung verwendete Phosgen wird vor Durchführung des erfindungsgemäßen Verfahrens auf eine Temperatur innerhalb des Bereichs von 200°C bis 600°C, vorzugsweise 250°C bis 500°C erhitzt.

Beide Eduktströme können vor der Mischung im Reaktor zur Stabilisierung der Strömung über drallerzeugende Einbauten geleitet werden.

Zur Durchführung des erfindungsgemäßen Verfahrens werden die vorerhitzten und gegebenenfalls drallbehafteten Ströme von Amin bzw. Amin-Inertgas-Gemisch einerseits und von Phosgen andererseits kontinuierlich einem Reaktionsraum ohne sich bewegende Teile zugeführt. Vor der Mischung der beiden Ströme wird mindestens einer der Ströme beschleunigt. Vorzugsweise ist dies der Phosgenstrom. Dabei wird das Maximum der Geschwindigkeit im Bereich von einem freien Strömungsdurchmessers vor bis einem freien Strömungsdurchmessers hinter der Mischstelle erreicht. Bevorzugt wird das Maximum der Geschwindigkeit an der Mischstelle erreicht.

Erfindungsgemäß wird ein Reaktor verwendet, welcher im Bereich der Zusammenführung der Reaktanden über eine verringerte Querschnittsfläche verfügt, die sich im Reaktionsteil wieder erweitert. Diese Konstellation erhöht die Gasgeschwindigkeit. Diese Verringerung der Querschnittsfläche mit nachfolgender Aufweitung folgt in einer bevorzugten Ausführungsform des erfindungsgemäßen Verfahrens der Form einer Venturi-Düse.

In einer Ausführungsform des erfindungsgemäßen Verfahrens weist der Reaktionsraum in Strömungsrichtung vor der Vermischung der beiden Edukte eine äußere Beheizung auf, durch die die Reaktanden erhitzt und durch die damit verbundene Volumenvergrößerung bei konstantem Strömungsquerschnitt auf eine höhere Geschwindigkeit beschleunigt werden.

Eine Kombination von Querschnittsverengung und äußerer Beheizung führt ebenfalls zum erfindungsgemäß günstigen Effekt.

Die Reaktoren bestehen im Allgemeinen aus Stahl, Glas, legiertem oder emaillierten Stahl und weisen eine Länge auf, die ausreichend ist, um unter den Verfahrensbedingungen eine vollständige Umsetzung des Amins mit dem Phosgen zu ermöglichen. Die Gasströme werden im Allgemeinen an einem Ende des Reaktionsraumes in diesen eingeleitet, wobei diese Einleitung beispielsweise durch an einem Ende des Reaktors angebrachte Düsen oder durch eine Kombination aus Düse und einem Ringspalt zwischen Düse und Wand erfolgen kann. Die Mischzone wird ebenfalls bei einer Temperatur innerhalb des Bereiches von 200°C und 600°C, vorzugsweise 250°C und 500°C gehalten, wobei diese Temperatur gegebenenfalls durch Beheizung des Reaktors aufrecht erhalten wird.

Bei der Durchführung des erfindungsgemäßen Verfahrens liegt im Allgemeinen der Druck in den Zuleitungen zum Reaktionsraum bei 200 mbar bis 3000 mbar und am Ausgang des Reaktionsraumes bei 150 mbar bis 2000 mbar, wobei durch Aufrechterhaltung eines geeigneten Differenzdruckes eine Strömungsgeschwindigkeit innerhalb des Reaktionsraumes von mindestens 3 m/s, vorzugsweise mindestens 6 m/s und besonders bevorzugt 10 m/s bis 120 m/s erreicht wird. Unter diesen Voraussetzungen herrschen innerhalb des Reaktionsraumes im Allgemeinen turbulente Strömungsverhältnisse vor.

Es ist ein Vorteil des erfindungsgemäßen Verfahrens, dass bei gleicher oder besserer Produktqualität eine höhere Raum-Zeit-Ausbeute des Reaktors erreicht wird, indem die Standzeit des Reaktors, d.h. die Produktionszeit im Verhältnis zur Stillstandszeit, die zur Reinigung des Reaktors erforderlich sind, je nach hergestelltem Isocyanat um 40-60 % erhöht werden kann.

### Beispiele

Das erfindungsgemäße Verfahren wird durch das nachfolgende Beispiel näher erläutert.

### Beispiel 1

In ein Mischrohr mit nachgeschalteter Diisocyanatkondensationsstufe und dieser nachfolgendem, mit Aktivkohle gefülltem Phosgen-Adsorptionsturm strömen kontinuierlich durch eine Düse mit einem Außendurchmesser von 1,7 mm, die in das Mischrohr hineinragt, 5,91 mol/h Phosgen, die in einem vorgeschalteten Wärmetauscher bei einem Druck von 1100 mbar auf eine Temperatur von 400°C erwärmt wurden. Durch den Ringspalt zwischen Düse und Mischrohr wird gleichzeitig ein auf 400°C erwärmtes Gemisch aus 1,26 mol gasförmigem Hexamethylendiamin und 1,25 mol Stickstoff stündlich in das Mischrohr geleitet. Der Durchmesser des Mischrohres variiert entlang der Längsachse, in dem er sich vor der Düse und bis 1,5 mm hinter der Düse mit einem Winkel von 10° bis auf 2,5 mm verringert und anschließend auf den nächsten 17,5 mm konstant bleibt (siehe Skizze des Reaktors). Dadurch befindet sich der außenströmende Aminstrom in einem beschleunigten Strömungszustand. Durch Anlegen eines Vakuums am Ausgang aus dem Phosgenadsorptionsturm wird eine Druck im Mischrohr von ca. 350 mbar aufrechterhalten. Das heiße, den Reaktionsraum verlassene Reaktionsgemisch wird in einer Kondensationsstufe durch Dichlorbenzol geleitet, welches bei einer Temperatur von 150-160°C gehalten wird. Hierbei erfolgt eine selektive Kondensation des gebildeten Diisocyanatohexans. Das die Waschstufe durchlaufende, im wesentlichen aus Stickstoff, Chlorwasserstoff und überschüssigem Phosgen bestehende Gasgemisch wird in dem Adsorptionsturm anschließend vom Phosgen befreit. Das Diisocyanat wird aus dem Waschlösungsmittel destillativ in reiner Form gewonnen. Die Ausbeute an 1,6-Diisocyanatohexan liegt bei 98,0% der Theorie.

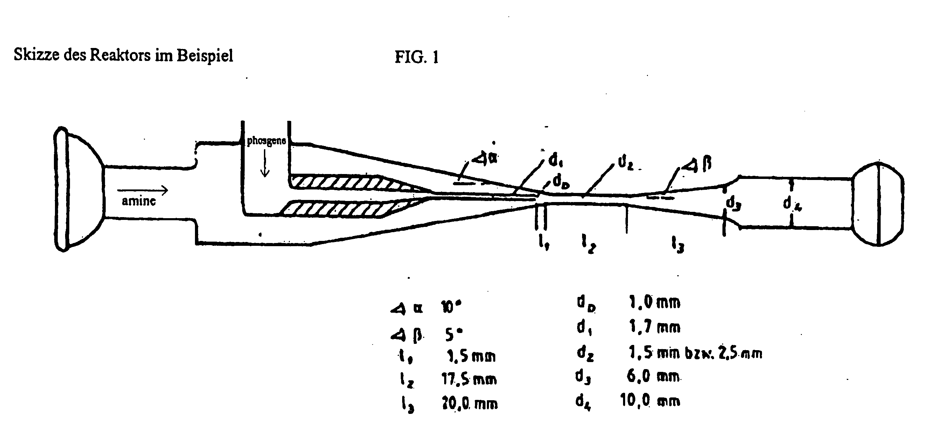

## Patentansprüche

1. Verfahren zur Herstellung von (cyclo)aliphatischen Diisocyanaten und Triisocyanaten der allgemeinen Formel (I)
R-(NCO)ₙ (I),
in welcher
R für einen (cyclo)aliphatischen Kohlenwasserstoffrest mit bis zu 15 Kohlenstoffatomen steht, mit der Maßgabe, dass zwischen zwei Aminogruppen mindestens zwei Kohlenstoffatome angeordnet sind, und n für die Zahl 2 oder 3 steht
durch Phosgenierung der entsprechenden Diamine oder Triamine der allgemeinen Formel (II)
R-(NH₂)ₙ (II),
in welcher
R und n die bei Formel (I) genannten Bedeutungen haben,
in der Gasphase, **dadurch gekennzeichnet, dass** die dampfförmigen Amine, gegebenenfalls verdünnt mit einem Inertgas oder mit den Dämpfen eines inerten Lösungsmittels, und Phosgen getrennt auf Temperaturen von 200°C bis 600°C erhitzt, mindestens einer der Eduktströme im Bereich der Mischung der Reaktanden beschleunigt und beide Reaktanden kontinuierlich in einem Reaktionsraum ohne sich bewegende Teile zur Reaktion gebracht werden, wobei ein Reaktor verwendet wird, der im Bereich der Mischung der Reaktanden über eine verringerte Querschnittsfläche verfügt, die sich im Reaktionsteil wieder erweitert.

2. Verfahren nach Anspruch 1, **dadurch gekennzeichnet, dass** die Ausgangsstoffe verdünnt und einem Inertgas und/oder mit Dämpfen eines inerten Lösungsmittels dem Reaktor zugeführt werden.

## Claims

1. Process for preparing (cyclo)aliphatic diisocyanates and triisocyanates of the general formula (I)
R-(NCO)ₙ (I),
wherein
R represents a (cyclo)aliphatic hydrocarbon radical having up to 15 carbon atoms, provided that there are at least two carbon atoms between two amino groups, and
n represents the number 2 or 3,
by phosgenating the corresponding diamines or triamines of the general formula (II)
R-(NH₂)ₙ (II),
wherein
R and n have the definition stated for formula (I),
in the gas phase, **characterized in that** the amines in vapor form, optionally diluted with an inert gas or with the vapors of an inert solvent, and phosgene are heated separately to temperatures of 200 °C to 600 °C, at least one of the reactant flows is accelerated in the region where the reactants are mixed, and both reactants are reacted continuously in a reaction chamber that does not have moving parts, wherein a reactor is used which has a cross-sectional area which is reduced in the region where the reactants are mixed, and widens again in the reaction section.

2. Process according to claim 1, **characterized in that** the starting materials are diluted and supplied to the reactor with an inert gas and / or with vapors of an inert solvent.

## Revendications

1. Procédé pour la préparation de diisocyanates et triisocyanates (cyclo)aliphatiques de formule générale (I)
R-(NCO)ₙ (I),
dans laquelle
R représente un radical hydrocarboné (cyclo)aliphatique contenant jusqu'à 15 atomes de carbone, sous réserve qu'entre deux groupes amino, il y a au moins deux atomes de carbone, et n est égal à 2 ou 3,
par phosgénation en phase gazeuse des diamines ou triamines correspondantes de formule générale (II)
R-(NH₂)ₙ (II),
dans laquelle
R et n ont les significations indiquées en référence à la formule (I),
**caractérisé en ce que** l'on chauffe séparément à des températures de 200 à 600 °C les amines à l'état de vapeurs, éventuellement diluées par un gaz inerte ou par les vapeurs d'un solvant inerte, et le phosgène, on accélère au moins un des courants de réactifs de départ dans la région du mélange des réactifs et on fait réagir les deux composants en continu dans une zone de réaction sans parties mobiles, dans laquelle l'on utilise un réacteur qui a une section réduite dans le domaine du mélange des réactifs, laquelle s'agrandit à nouveau dans la partie où se produit la réaction.

2. Procédé selon la revendication 1, **caractérisé en ce que** l'on envoie au réacteur les produits de départ dilués par un gaz inerte et / ou par les vapeurs d'un solvant inerte.
